# EUROPEAN PATENT SPECIFICATION

(11) **EP 3 209 738 B1**
(45) Date of publication and mention of the grant of the patent: **12.12.2018**
(21) Application number: 15791408.6
(22) Date of filing: 19.10.2015
(51) Int. Cl.: C09D 171/00, C08G 59/06, C08G 59/24, C09D 163/00, C09D 167/02, C08G 65/34, B65D 81/34

(54) **POLYETHERS DERIVED FROM BENZENE DIMETHANOL**
POLYETHER, DIE SICH VON BENZOLDIMETHANOL ABLEITEN
POLYÉTHERS DERIVÉS DE BENZÈNE DIMÉTHANOL

(30) Priority: 20.10.2014 US 201414518223
(43) Date of publication of application: 30.08.2017
(73) Proprietor: PPG Industries Ohio, Inc., Cleveland, OH 44111 (US)
(72) Inventor: KALEEM, Kareem, Loveland, Ohio 45140 (US); MOUSSA, Youssef, Loveland, Ohio 45140 (US)
(74) Representative: f & e patent
(86) International application number: PCT/US2015/056139
(87) International publication number: WO 2016/064696

(56) References cited:
- WO-A1-01/32790
- WO-A1-2012/151184
- WO-A2-2011/130671
- WO-A2-2012/109278
- US-A1- 2004 132 895

## Description

### FIELD OF THE INVENTION

The present invention relates to polyethers derived from benzene dimethanol and to food-contacting containers coated with compositions containing the polyethers.

### BACKGROUND OF THE INVENTION

A wide variety of coatings have been used to coat the surfaces of food and beverage containers. For example, metal cans are sometimes coated using coil coating or sheet coating operations, that is, a coil or sheet of steel or aluminum, is coated with a suitable composition and cured. The coated substrate is then formed into the can body or can end. Alternatively, the coating composition may be applied, for example, by spraying and dipping, to the formed can and then cured. Coatings for food and beverage containers should preferably be capable of high speed application to the substrate and provide the necessary properties when cured to perform in a demanding end use environment. For example, the coating should be safe for food contact and have excellent adhesion to the substrate.

Many of the coating compositions for food and beverage containers are based on polyether resins that are based on polyglycidyl ethers of bisphenol A. Bisphenol A in container coatings either as bisphenol A itself (BPA) or derivatives thereof, such as diglycidyl ethers of bisphenol A (BADGE), epoxy novolak resins and polyols prepared with bisphenol A and bisphenol F are problematic. Although the balance of scientific evidence available to date indicates that small trace amounts of BPA or BADGE that might be released from existing coatings does not pose health risks to humans, these compounds are nevertheless perceived by some as being harmful to human health. Consequently, there is a strong desire to eliminate these compounds from coatings for food and beverage containers. Accordingly, what are desired are container coating compositions for food and beverage containers that do not contain extractable quantities of BPA, BADGE or other derivatives of BPA and yet have commercially acceptable properties.

WO2011130671 discloses a container comprising a food-contacting surface coated with a composition comprising a polyether composition derived from aromatic phenols or cyclic diols by functionalization with hydroxy phenyl acetic acid and diglycidyl ethers.

### SUMMARY OF THE INVENTION

The present invention provides a container comprising a food-contacting surface wherein at least a portion of the food-contacting surface is coated with a polyether composition derived from benzene dimethanol containing at least one of the following segments: 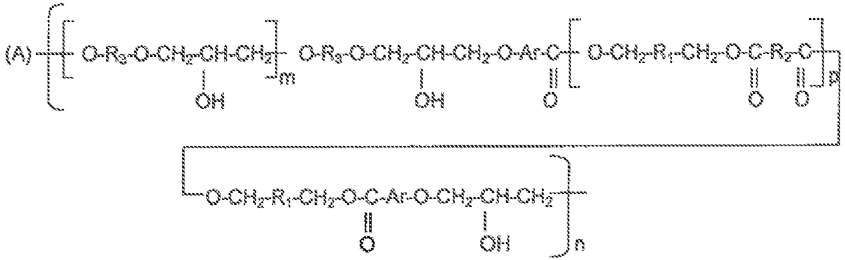 where R₁ is phenylene; R₂ is a saturated aliphatic radical, R₃ is a (cyclo)aliphatic radical, an arylene radical or an alkarylene radical, m is 0 to 12, n is 1 to 12 and p is less than 5. or 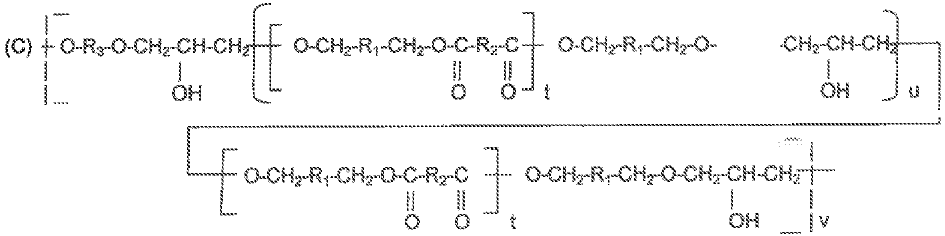 where R₁ is phenylene, R₂ is a saturated aliphatic radical, R₃ is a (cyclo)aliphatic radical, an arylene radical or an alkarylene radical, t is less than 5, u is 0 to 12 and v is 1 to 12.

The invention also provides for a container comprising a food-contacting surface wherein at least a portion of the food-contacting surface is coated with a composition comprising the polyethers of (A) and/or (C) grafted to a (meth)acrylic polymer.

### DETAILED DESCRIPTION

As used herein, unless otherwise expressly specified, all numbers such as those expressing values, ranges, amounts or percentages may be read as if prefaced by the word "about", even if the term does not expressly appear. Moreover, it should be noted that plural terms and/or phrases encompass their singular equivalents and vice versa. For example, "a" polymer, "a" crosslinker, and any other component refers to one or more of these components.

When referring to any numerical range of values, such ranges are understood to include each and every number and/or fraction between the stated range minimum and maximum.

As employed herein, the term "polyol" or variations thereof refers broadly to a material having an average of two or more hydroxyl groups per molecule. The term "polycarboxylic acid" refers to the acids and functional derivatives thereof, including anhydride derivatives where they exist, and lower alkyl esters having 1-4 carbon atoms.

As used herein, the term "polymer" refers broadly to prepolymers, oligomers and both homopolymers and copolymers. The term "resin" is used interchangeably with "polymer".

The terms "acrylic" and "acrylate" are used interchangeably (unless to do so would alter the intended meaning) and include acrylic acids, anhydrides, and derivatives thereof, such as their C₁-C₅ alkyl esters, lower alkyl-substituted acrylic acids, e.g., C₁-C₂ substituted acrylic acids, such as methacrylic acid, ethacrylic acid, etc., and their C₁-C₅ alkyl esters, unless clearly indicated otherwise. The terms "(meth)acrylic" or "(meth)acrylate" are intended to cover both the acrylic/acrylate and methacrylic/methacrylate forms of the indicated material, e.g., a (meth)acrylate monomer. The term "acrylic polymer" refers to polymers prepared from one or more acrylic monomers.

(Cyclo)aliphatic refers to both cyclic aliphatic compounds and to straight chain aliphatic compounds.

Carboxylic acid refers to both acid and equivalent functionality such lower alkyl esters [C(1)-C(4)] thereof.

As used herein, "a" and "the at least one" and "one or more" are used interchangeably. Thus, for example, a coating composition that comprises "a" polymer can be interpreted to mean the coating composition includes "one or more" polymers.

As used herein, the molecular weights are determined by gel permeation chromatography using a polystyrene standard. Unless otherwise indicated, molecular weights are on a number average basis (Mn).

By "food" in "food-contacting surface" is meant to include solid foodstuffs as well as beverages.

The polyethers of the invention are derived from benzene dimethanol:

HO-CH₂-R₁-CH₂-OH

where R₁ is phenylene.

The benzene dimethanol can be modified by a subsequent reaction with a hydroxy aromatic carboxylic acid such as 4-hydroxy benzoic acid to form a phenolic-terminated adduct. 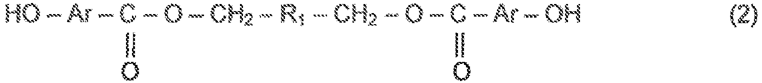 where Ar is arylene.

The phenolic adduct (2) can further be reacted with a diglycidyl ether such as: 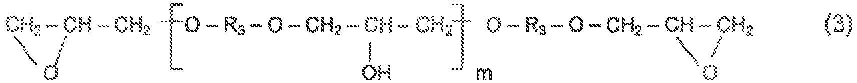 where R₃ is a divalent (cyclo)aliphatic radical; an arylene radical or an alkarylene radical, and m is 0 to 12, to form a polyether having the following structure: 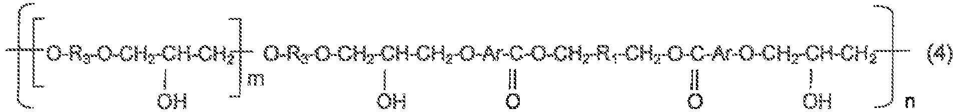 where m is 0 to 12 and n is 1 to 12.

The diglycidyl ethers can be diglycidyl ethers of (cyclo)aliphatic diols, arylene diols or alkarylene diols including mixed diols. Examples of such diols include 1,4-butanediol, cyclohexane dimethanol, resorcinol, hydroquinone and catechol. An example of an alkaryl diol is alkyl-substituted catechol such as t-butyl catechol.

To enhance the flexibility of the polyether, the polyethers can be modified with a dicarboxylic acid such as a saturated aliphatic dicarboxylic acid containing from 4 to 12 carbon atoms, such as a linear alkylene dicarboxylic acid, for example, adipic acid and/or sebacic acid. An adduct or an oligomeric polyester can be prepared from the dicarboxylic acid and the benzene dimethanol to form a hydroxy-terminated adduct or oligomer that could be further reacted with the hydroxy aromatic carboxylic acid or the lower alkyl ester thereof to give a material having the following segments: 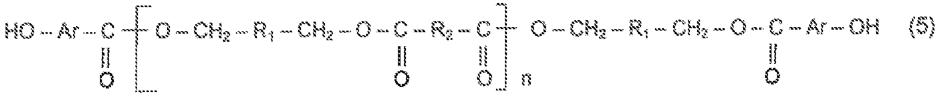 where Ar is arylene; R₁ is phenylene; R₂ is a divalent saturated aliphatic group and n is less than 5.

In the above structure (5), n is 0 when no polyester is prepared; with a polyester, n is typically 1 to 2.

The modified diphenolic compound (5) could then be reacted with a diglycidyl ether (3 above) to form a polyether having the following segments: 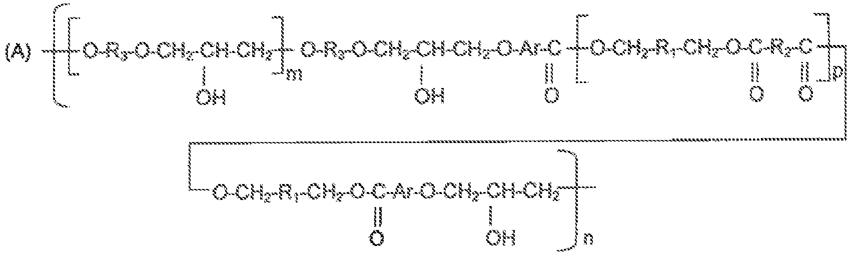 where R₁ is phenylene; R₂ is a saturated aliphatic radical, R₃ is a (cyclo)aliphatic radical, an arylene radical or an alkarylene radical, m is 0 to 12, n is 1 to 12 and p is less than 5. In the above structure, p is 0 when no polyester is prepared; with a polyester, p is typically 1 to 2.

The polyether (A) may be terminated with epoxy groups or phenolic hydroxyl groups such as polyethers of the following structures: 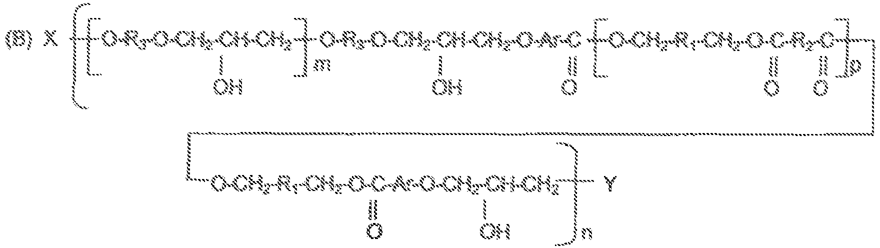 where X is 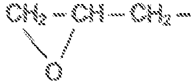 or 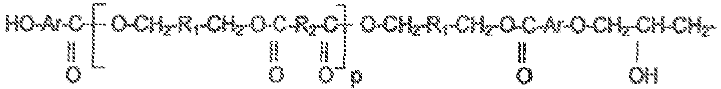 and where Y is 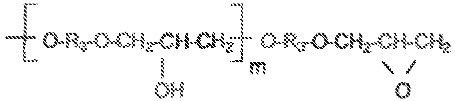 or 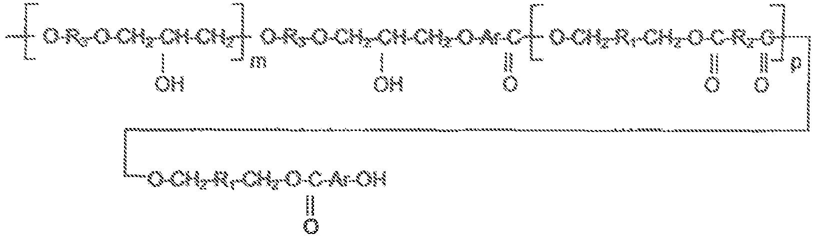 The identity of X and Y will depend on the equivalent ratio of (2) to (3). Typically the equivalent ratio of (2) to (3) is from 0.8-1.2:1.

Modification of the benzene dimethanol or the polyester thereof with the hydroxy aromatic carboxylic acid or ester thereof to prepare the polyether depicted by (5) above is done by traditional esterification techniques such as heating the benzene dimethanol with the hydroxy aromatic carboxylic acid or ester thereof typically in the presence of a tin catalyst while distilling off the water or alcohol depending on whether the hydroxy aromatic carboxylic acid or its lower alkyl ester is used. The hydroxy aromatic carboxylic acid is typically used in an equivalent excess, i.e., the acid equivalents of the hydroxy aromatic carboxylic acid to the hydroxy equivalents of the polyester is greater than 1, to provide phenolic hydroxyl groups for further reaction with the diglycidyl ether.

The reaction conditions for reaction of the diphenolic compound (5) with the diglycidyl ether (3) are conditions typically used for diphenolic advancement of diglycidyl ether. The reaction is conducted in organic solvent such as a ketone and in the presence of catalyst such as an onium compound at elevated temperatures such as 100 to 160°C. and for a time sufficient to achieve the desired epoxy equivalent weight, typically 30 minutes to 3 hours.

For the more flexible polyethers, a polyester adduct or oligomer is prepared from reacting benzene dimethanol with a dicarboxylic acid anhydride to prepare the adduct or oligomeric product having segments of the following structure: 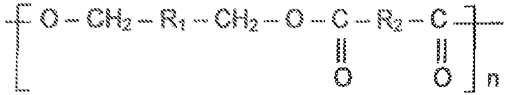 where R₁ is phenylene and R₂ is a saturated aliphatic radical such as a saturated alkylene radical containing from 4 to 12 carbon atoms; n is less than 5 such as 1 to 2. The reaction conditions for preparing the adduct or oligomeric polyester are those typically used in polyester synthesis. The polyesters are prepared by condensation polymerization conditions as generally described by Zeno Wicks, Jr. et al. in Organic Coatings Science and Technology, Vol. 1, pages 122-132 (John Wiley & Sons; New York, 1992).

Besides the polyethers described above, polyethers can be prepared by reacting benzene dimethanol with epichlorohydrin to form a diglycidyl ether having the following structure: 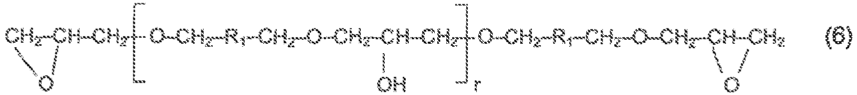 where r is from 0 to 12.

The diglycidyl ether (6) can be advanced with a (cyclo)aliphatic diol or a dihydric phenol.

HO-R₃-OH (7)

where R₃ is a (cyclo)aliphatic radical, an arylene radical or an alkarylene radical. to ring open the epoxide group to form a polyether with the following structure: 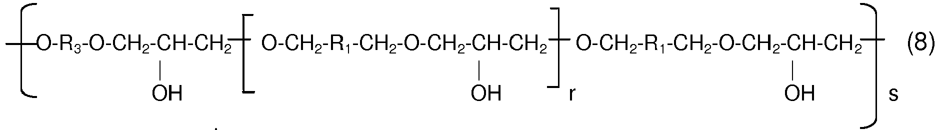 where r is 0 to 12 and s is 1 to 12.

When modified with a dicarboxylic anhydride to form the polyester adduct or oligomer with the benzene dimethanol as generally described above and further reaction with epichlorohydrin would produce a diglycidyl ether having the following structure: 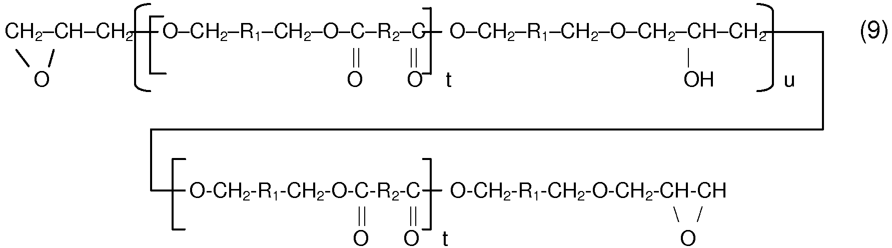 where R₂ is a saturated aliphatic radical, t is less than 5, such as 1 to 2, and u is 0 to 12.

The diglycidyl ether (9) can be advanced or chain extended with a (cyclo)aliphatic diol or a dihydric phenol (7) that ring opens the epoxide groups to give a polyether having the following structure: 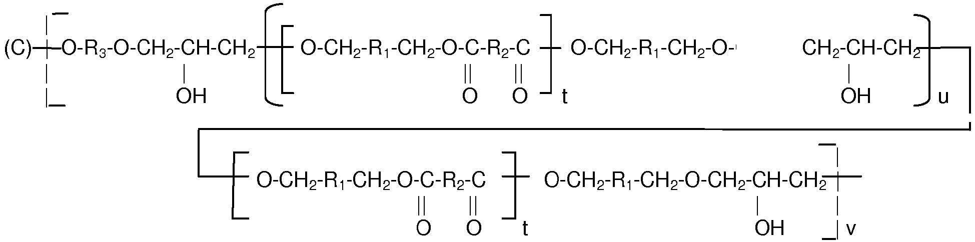 where R₁ is phenylene, R₂ is a saturated alphatic radical, R₃ is a (cyclo)aliphatic radical, an arylene radical or an alkarylene radical, t is less than 5, u is 0 to 12 and v is 1 to 12. In the above structure, t is 0 when no polyester is prepared. With a polyester, t is typically 1 to 2.

Examples of suitable chain-extenders are 1,4-butanediol, cyclohexane dimethanol, resorcinol, hydroquinone, catechol and t-butyl catechol.

The polyether (C) may be terminated with epoxy groups or OH groups such as polyethers of the following structures: 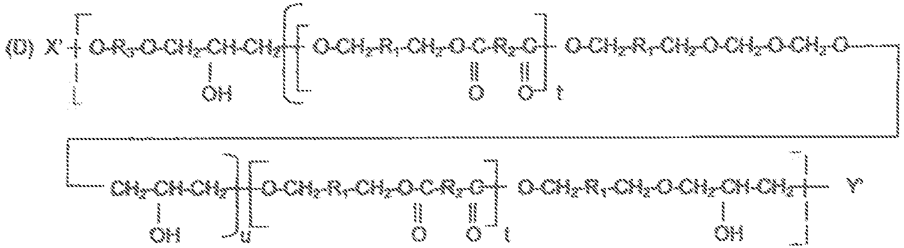 where X' is H or 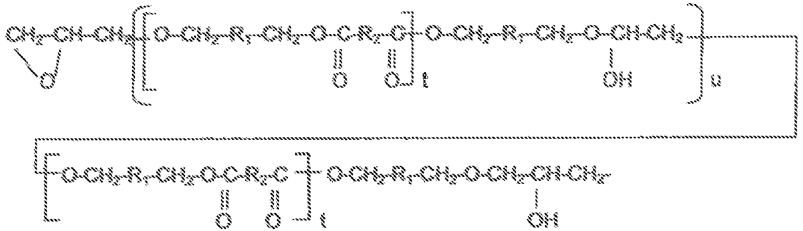 and Y' is O-R₃-OH, or

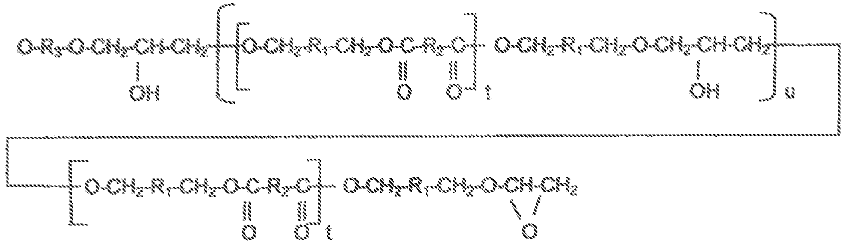

Reaction conditions for reacting the benzene dimethanol or the polyester adduct or oligomer thereof with epichlorohydrin can be those generally described in US 3,041,300. For example, in one-liter flask equipped with stirrer, reflux condenser and thermometer, one mole (138 gms) of benzene dimethanol (BDM), 3 moles of epichlorohydrin (277.5 gms) and 50% aqueous sodium hydroxide (160 gms, 2 moles) can be heated to specific temperature (e.g. 90-95°C.). After holding at reaction temperature, the reaction mixture can be heated to remove water to complete the reaction using Dean Stark apparatus. The resultant mixture would be washed with water to remove sodium chloride. The water insoluble layer will be extracted with methylene chloride and dried over anhydrous sodium sulfate. The diglycidyl ether can be recovered after removal of methylene chloride by vacuum distillation.

Advancement or chain extension reactions mentioned above can be conducted under conditions typically used for advancement of diglycidyl ethers. The reaction is conducted in organic solvent such as a ketone and in the presence of a catalyst such as an onium compound at elevated temperatures such as 100 to 160°C. and for a time to achieve the desired epoxy equivalent weight, typically 30 minutes to 3 hours.

The polyethers of the invention, that those have the segments (A) and (C) are applied to the substrate as a component in a coating composition that includes a liquid carrier. The liquid carrier may be water, organic solvent or mixtures thereof. Accordingly, the liquid coating compositions of the present invention may be aqueous based (containing water and optionally some water-miscible organic solvent) or be organic solvent based, that is, substantially no water (i.e., less than 2% by weight water based on total weight of the coating composition). Examples of suitable organic solvents are glycol ethers, alcohols, aromatic or aliphatic hydrocarbons, ketones, esters and mixtures thereof. The liquid carriers are selected to provide a dispersion or solution of the polyether for further coating formulation.

The polyethers can be dissolved in an organic solvent and formulated with a crosslinking agent such as an aminoplast or phenolplast curing agent (as described below) and normal additives known in coatings for food-contacting surfaces of containers. Typically, organic solvent-based coating compositions based on the polyethers have resin solids contents of 20 to 50% by weight based on total weight of the coating composition. The polyethers may also be formulated in aqueous-based coating compositions, for example, the polyether can be prepared with terminal 1,2-epoxy functionality that can be reacted with phosphoric acid and at least partially neutralized with a base to form phosphate salt groups making the polyether dispersible in an aqueous carrier.

Because of the activated methylene group in benzene dimethanol, the polyether can be grafted via proton abstraction to a water-dispersible (meth)acrylic polymer such as by grafting with a (meth)acrylic acid functional monomer that contains double bonds, which is polymerizable by a free radical mechanism. Examples of such monomers are (meth)acrylic acid and ethylenically unsaturated monomers not containing acid groups such as (meth)acrylic acid esters, styrene and the like. The resulting graft copolymer can then be at least partially neutralized with a base such as a tertiary amine. The resin solids content of the aqueous-based coating compositions is typically from 20 to 50% by weight resin solids based on total weight of the coating composition.

The acrylic portion of the polyether-(meth)acrylic graft copolymer comprises polymerized ethylenically unsaturated monomers which include carboxyl functional monomers such as (meth)acrylic acid and unsaturated dicarboxylic acids such as maleic or fumaric, to provide carboxyl functional for dispersing the polyether-(meth)acrylic copolymer mixture into water. The balance of the monomers preferably are non-functional under the contemplated conditions of polymerization, although small amounts of other reactive monomers may be used such as hydroxy monomers illustrated by 2-hydroxy ethyl (meth)aclylate, amide monomers illustrated by (meth)acrylamide, or N-methylol monomers illustrated by N-methylol (meth)acrylamide. The remaining monomers are non-functional but copolymerizable ethylenic monomers illustrated by (meth)acrylate esters, such as ethyl (meth)acrylate, methyl (meth)acrylate or isobutyl (meth)acrylate, vinyl aromatic compounds, styrene, or vinyl toluene, vinyl acetate, vinyl chloride, vinylidene chloride and other ethylenically unsaturated vinyl monomers such as butadiene and (meth)acrylonitrile. The (meth)acrylic polymer component comprises by weight between about 5% and 40% based on the weight of the (meth)acrylic grafted polyether.

The polyether-acrylic graft copolymer may be prepared by non-aqueous polymerization of the ethylene monomers with the polyether resin. The polyether resin can be heated in a reactor wherein the polymerizable monomer can be added slowly over a period of at least two or three hours along with a solvent and a free radical initiator. Although the reaction may be conducted in the absence of a solvent, some solvent is desirable for the polymerization of monomers in the presence of polyether resin. Solvents such as xylene, benzene, ethyl benzene, toluene, and the alkoxy alkanols are satisfactory. Alcohols such as methanol, ethanol, propanol, butanol, and the like, are suitable, with ethylene glycol monobutyl ether and butanol being preferred. Ethylene glycol monobutyl ether, ethylene glycol monohexyl ether, diethylene glycol monobutyl ether, and the like are most suitable. For subsequent dispersion into water, the solvents selected are usually water-soluble materials, such as butanol, propanol, ethylene glycol monoethyl ether, and the like, although small amounts of mineral spirits, hexane, and similar aliphatics may be used.

As mentioned above, the coating compositions of the present invention contain a crosslinking agent. Examples of crosslinking agents are phenolplast and aminoplast.

Suitable phenolplast resins include the condensation products of aldehydes with phenols. Formaldehyde and acetaldehyde are preferred aldehydes. Various phenols can be employed such as phenol, cresol, p-phenylphenol, p-tertbutylphenol, p-tert-amylphenol, and cyclopentylphenol.

Suitable aminoplast resins are the condensation products of aldehydes such as formaldehyde, acetaldehyde, crotonaldehyde, and benzaldehyde with amino- or amido-group-containing substances such as urea, melamine, and benzoguanamine. Examples of suitable aminoplast crosslinking resins include, without limitation, benzoguanamine-formaldehyde resins, melamine-formaldehyde resins, etherified melamine-formaldehyde, and urea-formaldehyde resins.

The level of curing agent (e.g., crosslinker) used will typically depend on the type of curing agent, the time and temperature of the bake, the molecular weight of the binder polymer, and the desired coating properties. If used, the crosslinker is typically present in an amount of up to 50 wt-%, preferably up to 30 wt-%, and more preferably up to 15 wt-%. If used, a crosslinker is preferably present in an amount of at least 0.1 wt-%, more preferably at least 1 wt-%, and even more preferably at least 1.5 wt-%. These weight percentages are based upon the total weight of the resin solids in the coating composition.

The coating composition of the present invention may also include other optional polymers that do not adversely affect the coating composition or a cured coating composition resulting therefrom. Such optional polymers are typically included in a coating composition as a filler material, although they can also be included, for example, as a binder polymer, a crosslinking material, or to provide desirable properties. One or more optional polymers (e.g., filler polymers) can be included in a sufficient amount to serve an intended purpose, but not in such an amount to adversely affect a coating composition or a cured coating composition resulting therefrom.

Such additional polymeric materials can be nonreactive, and hence, simply function as filters. Such optional nonreactive filler polymers include, for example, polyesters and (meth)acrylic polymers. Alternatively, such additional polymeric materials or monomers can be reactive with other components of the composition. If desired, reactive polymers can be incorporated into the compositions of the present invention, to provide additional functionally for various purposes, including crosslinking or dispersing the polymer of the present invention into water. Examples of such reactive polymers indude, for example, functionalized polyesters and functionalized (meth)acrylic polymers.

Another optional ingredient is a catalyst to increase the rate of cure. Examples of catalysts include, but are not limited to, strong acids, e.g., phosphoric acid, dodecylbenzene sulphonic acid (DDBSA), available as CYCAT 600 from Cytec, methane sulfonic acid (MSA), p-toluene sulfonic acid (pTSA), dinonylnaphthalene disulfonic acid (DNNDSA). If used a catalyst is preferably present in an amount of at least 0.01 wt-%, such as at least 0.1 wt-%, based on the weight of nonvolatile material in the coating composition. If used, a catalyst is preferably present in an amount of no greater than 3 wt-%, such as no greater than 1 wt-%, based on the weight of nonvolatile material in the coating composition.

Another useful optional ingredient is a lubricant (e.g., a wax), which facilitates manufacture of fabricated metal articles (e.g., closures and food or beverage can ends) by imparting lubricity to sheets of coated metal substrate. Non-limiting examples of suitable lubricants include, for example, natural waxes such as Carnauba wax or lanolin wax, polytetrafluoroethane (PTFE) and polyethylene-type lubricants. If used, a lubricant is preferably present in the coating composition in an amount of at least 0.1 wt-%, such as no greater than 2 wt-%, and typically no greater than 1 wt-%, based on the total weight of nonvolatile material in the coating composition.

Another useful optional ingredient is a pigment, such as titanium dioxide. If used, a pigment is present in the coating composition in an amount of no greater than 70 wt-%, such as no greater than 50 wt-%, and typically no greater than 40 wt-%, based on the total weight of solids in the coating composition.

Surfactants can be optionally added to the coating composition, e.g., to aid in flow and wetting of the substrate. Examples of surfactants include, but are not limited to, nonylphenol polyethers and salts and similar surfactants known to persons skilled in the art. If used, a surfactant is preferably present in an amount of at least 0.01 wt-%, such as at least 0.1 wt-%, based on the weight of resin solids. If used, a surfactant is usually present in an amount no greater than 10 wt-%, and typically no greater than 5 wt-%, based on the weight of resin solids.

The coating compositions used in the practice of this invention are substantially free, may be essentially free and/or may be completely free of bisphenol A and derivatives or residues thereof, including bisphenol A and bisphenol A diglycidyl ether ("BADGE"). A reaction product and/or coating that is substantially bisphenol A free is sometimes referred to as "BPA non intent" because BPA, including derivatives or residues thereof, are not intentionally added but may be present in trace amounts such as because of impurities or unavoidable contamination from the environment The reaction product and/or costings of the present invention can also be substantially free, essentially free and/or completely free of bisphenol F and derivatives or residues thereof, including bisphenol F and bisphenol F diglycidyl ether ("BPFDG"). The term "substantially free" as used in this context means the reaction product and/or coating compositions contain less than 1000 parts per million (ppm), "essentially free" means less than 100 ppm and "completely free" means less than 20 parts per billion (ppb) of any of the above compounds or derivatives or residues thereof.

The coating composition of the present invention can be present as a layer of a mono-layer coating system or one or more layers of a multi-layer coating system. The coating composition can be used as a primer coat, an intermediate coat, a top coat, or a combination thereof. The coating thickness of a particular layer and the overall coating system will vary depending upon the coating material used, the substrate, the costing application method, and the end use for the coated article. Mono-layer or multi-layer coating systems including one or more layers formed from a coating composition of the present invention may have any suitable overall coating thickness, but will typically have an overall average dry coating thickness of from about 1 to about 60 microns and more, typically from about 2 to about 15 microns. Typically, the average total coating thickness for rigid metal food or beverage can applications will be about 3 to about 10 microns. Coating systems for closure applications may have an average total coating thickness up to about 15 microns. In certain embodiments in which the coating composition is used as an interior coating on a drum (eg., a drum for use with food or beverage products), the total coating thickness may be approximately 25 microns.

The coating composition of the present invention may be applied to a substrate either prior to, or after, the substrate is formed into an article (such as, for example, a food or beverage container or a portion thereof). In one embodiment, a method is provided that includes: applying a coating composition described herein to a metal substrate (e.g., applying the composition to the metal substrate in the form of a planar coil or sheet), curing the composition, and forming (e.g., via stamping) the substrate into a packaging container or a portion thereof (e.g., a food or beverage can or a portion thereof). For example, riveted beverage can ends having a cured coating of the present invention on a surface thereof can be formed in such a process. In another embodiment, the coating composition is applied to a preformed metal food or beverage can, or a portion thereof. For example, in some embodiments, the coating composition is spray applied to an interior surface of a preformed food or beverage can (e.g., as typically occurs with "two-piece" food or beverage cans). After applying the coating composition onto a substrate, the composition can be cured using a variety of processes, including, for example, oven baking by either conventional or convectional methods, or any other method that provides an elevated temperature suitable for curing the coating. The curing process may be performed in either discrete or combined steps. For example, substrates can be dried at ambient temperature to leave the coating compositions in a largely un-crosslinked state. The coated substrates can then be heated to fully cure the compositions. In certain instances, coating compositions of the present invention can be dried and cured in one step.

The cure conditions will vary depending upon the method of application and the intended end use. The curing process may be performed at any suitable temperature, including, for example, oven temperatures in the range of from about 100°C. to about 300°C., and more typically from about 177°C. to about 250°C. If metal coil is the substrate to be coated, curing of the applied coating composition may be conducted, for example, by heating the coated metal substrate over a suitable time period to a peak metal temperature ("PMT") of preferably greater than about 350°F. (177°C.). More preferably, the coated metal coil is heated for a suitable time period (e.g., about 5 to 900 seconds, more typically about 5 to 30 seconds) to a PMT of at least about 425°F. (218°C.).

The coating compositions of the present invention are particularly useful for coating metal substrates. The coating compositions may be used to coat packaging articles such as a food or beverage container, or a portion thereof. In preferred embodiments, the container is a food or beverage can and the surface of the container is the surface of a metal substrate. The polymer can be applied to a metal substrate either before or after the substrate is formed into a can (e.g., two-piece cans, three-piece cans) or portions; thereof, whether it be a can end or can body. Polymers of the present invention are suitable for use in food-contact situations and may be used on the inside of such cans. They are particularly useful on the interior of two-piece or three-piece can ends or bodies.

The metal substrate used in forming rigid food or beverage cans, or portions thereof, typically has a thickness in the range of about 0.005 inches to about 0.025 inches. Electro tinplated steel, cold-rolled steel, and aluminum are commonly used as metal substrates for food or beverage cans, or portions thereof. In embodiments in which a metal foil substrate is employed in forming, e.g., a packaging article, the thickness of the metal foil substrate may be even thinner than that described above.

The coating compositions of the present invention may be suitable, for example, for spray coating, coil coating, wash coating, sheet coating, and side seam coating (eg., food can side seam coating). A further discussion of such application methods is provided below. It is contemplated that coating compositions of the present invention may be suitably used in each of these application methods discussed further below, including the end uses associated therewith.

Spray coating includes the introduction of the coating composition into the inside of a preformed packaging container. Typical preformed packaging containers suitable for spray coating include food cans, beer and beverage containers, and the like. The spray process preferably utilizes a spray nozzle capable of uniformly coating the inside of the preformed packaging container. The sprayed performed container is then subjected to heat to remove any residual carriers (e.g., water or solvents) and harden the coating.

A coil coating is described as the coating of a continuous coil composed of a metal (e.g., steel or aluminum). Once coated, the coating coil is subjected to a short thermal curing cycle, for hardening (e.g., drying and curing) of the coating. Coil coatings provide coated metal (e.g., steel and/or aluminum) substrates that can be fabricated into formed articles, such as two-piece drawn food cans, three-piece food cans, food can ends, drawn and ironed cans, beverage can ends, and the like.

A wash coating is commercially described as the coating of the exterior of two-piece drawn and ironed ("D&I") cans with a thin layer of protectant coating. The exterior of these D&I cans are "wash-coated" by passing pre-formed two-pieoe D&I cans under a curtain of a coating composition. The cans are inverted, that is, the open end of the can is in the "down" position when passing through the curtain. This curtain of costing composition takes on a "waterfall-like" appearance. Once these cans pass under this curtain of coating composition, the liquid coating material effectively coats the exterior of each can. Excess coating is removed through the use of an "air knife". Once the desired amount of coating is applied to the exterior of each can, each can is passed through a thermal, ultraviolet, and/or electromagnetic curing oven to harden (e.g., dry and cure) the coating. The residence time of the coated can within the confines of the curing oven is typically from 1 minute to 5 minutes. The curing temperature within this oven will typically range from 150°C. to 220°C.

A sheet coating is described as the coating of separate pieces of a variety of materials (e.g., steel or aluminum) that have been pre-cut into square or rectangular "sheets". Typical dimensions of these sheets are approximately one square meter. Once coated, the coating is hardened (e.g., dried and cured) and the coated sheets are collected and prepared for subsequent fabrication. Sheet coatings provide coated metal (e.g., steel or aluminum) substrates that can be successfully fabricated into formed articles, such as two-piece drawn food cans, three-piece food cans, food can ends, drawn and ironed cans, beverage can ends (including, e.g., riveted beverage can ends having a rivet for attaching a pull tab thereto), and the like.

A side seam coating is described as the application of a powder coating or the spray application of a liquid coating over the welded area of formed three-piece food cans. When three-piece food cans are being prepared, a rectangular piece of coated substrate is formed into a cylinder. The formation of the cylinder is rendered permanent due to the welding of each side of the rectangle via thermal welding. Once welded, each can typically requires a layer of coating, which protects the exposed "weld" from subsequent corrosion or other effects to the contained foodstuff. The coatings that function in this role are termed "side seam stripes". Typical side seam stapes are spray applied and cured quickly via residual heat from the welding operation in addition to a small thermal cure in an oven.

### EXAMPLES

The following examples are offered to aid in understanding of the present invention and are not to be construed as limiting the scope thereof. Unless otherwise indicated, all parts and percentages are by weight.

### EXAMPLE 1

### Diphenolic Adduct of Benzene Dimethanol and Methyl 4-Hydroxy Benzoate

In a 4-neck flask equipped with stirrer, thermometer, packed column with head temperature, water condenser and nitrogen inlet, charge 106.4 parts of Methyl 4-hydroxy benzoate (MHBA, Sigma-Aldrich) and 96.6 grams of 1,4-Benzene dimethanol (BDM, Ark Pharm, Inc.). 0.6 grams of Butyl Titanate catalyst (Sigma-Aldrich) was added to effect transesterification. The mixture was heated slowly to 180°C. (356°F.). As the distillate started to collect, increase batch temperature accordingly to maximum reactor temperature of 220°C. (428°F.) not allowing column temperature to exceed 65°C. Maintain steady rate of distillation to remove methanol continuously. Process until material no more methanol is collected. 70-75% of theoretical methanol was collected. The batch was cooled to 200°C. and poured into glass container. The brittle material was washed with methanol and dried to remove residual methanol.

### EXAMPLE 2

### Diphenolic Adduct of BDM and 4-Hydroxy Benzoic Acid

In a 4-neck flask equipped with stirrer, thermometer, Dean-Stark apparatus with xylene., water condenser and nitrogen inlet, charge 211 parts of 4-Hydroxy benzoic acid (HBA, Sigma-Aldrich),105.5 grams of 1,4-Benzene dimethanol (BDM, Ark Pharm, Inc.) and 34 parts of Xylene. 0.2 grams of Fascat 2003 catalyst was added to effect esterification. The mixture was heated slowly to 160°C, As the distillate started to collect, increase batch temperature accordingly to maximum reactor temperature of 215°C. Maintain steady rate of reflux to remove water continuously. Process until material no more water is collected. 80% of theoretical water was collected. The batch was cooled to 200°C. and poured into glass container. The brittle material was washed with methanol and dried.

### EXAMPLE 3

### Polyether from Advancing Cyclohexane Diglycidyl Ether with Diphenolic Adduct of Benzene Dimethanol and Methyl 4-Hydroxy Benzoic Acid

In a 4-neck flask equipped with stirrer, thermometer, water condenser and nitrogen inlet, charge 60 parts of above diphenolic adduct (BDM/MHBA), 62.54 grams of CHDMDGE (EEW ∼ 159, Erisys 22S), 21 parts of MIBK and 0.6 grams of Ethyl triphenyl phosphonium iodide catalyst (Sigma-Aldrich). The mixture was heated slowly to 115°C. (239°F.) and the mixture turned clear. Continue to heat to 155°C. and mixture started to reflux heavily. Hold the batch for one hour and mixture became viscous. After one hour hold, heat was turned off and 120 parts of Butyl Cellosolve solvent was added to obtain 45.6% solids resin solution. The Epoxy Equivalent Weight of final polyether resin was determined to be ∼ 816 based on solids. The resin was drawn down on metal tin plate and baked at 400°F. (204°C.) for 10 minutes and was tack free.

Whereas particular embodiments of this invention have been described above for purposes of illustration, it will be evident to those skilled in the art that numerous variations of the details of the present invention may be made without departing from the invention as defined in the appended claims.

Although various embodiments of the invention have been described in terms of "comprising", embodiments consisting essentially of or consisting of are also within the scope of the present invention.

## Claims

1. A container comprising a food-contacting surface wherein at least a portion of the food-contacting surface is coated with a composition comprising a polyether composition derived from benzene dimethanol containing at least one of the following segments: 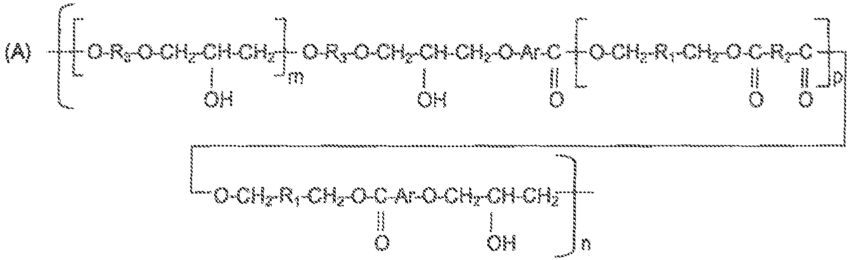 where R₁ is phenylene; R₂ is a saturated aliphatic radical, R₃ is a (cyclo)aliphatic radical, an arylene radical or an alkarylene radical, m is 0 to 12, n is 1 to 12 and p is less than 5, or 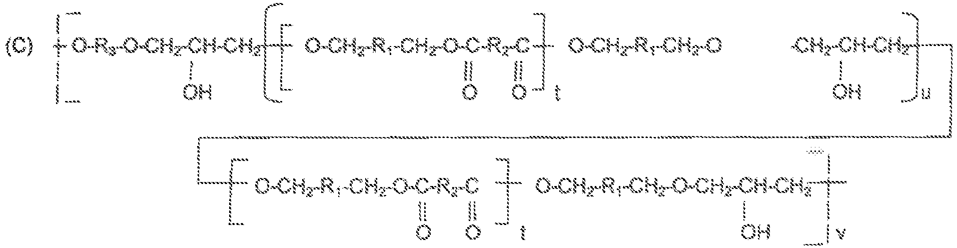 where R₁ is phenylene, R₂ is a saturated aliphatic radical, R₃ is a (cyclo)aliphatic radical, an arylene radical or an alkarylene radical, t is less than 5, u is 0 to 12 and v is 1 to 12.

2. The container of claim 1 in which (A) is terminated with epoxy groups or phenolic hydroxyl groups.

3. The container of claim 1 in which (C) is terminated with epoxy groups or phenolic hydroxyl groups.

4. The container of claim 1 where Ar is phenylene.

5. The container of claim 1 where R₂ is a linear alkylene radical containing from 4 to 12 carbon atoms.

6. The container of claim 1 where p is 1 to 2.

7. The container of claim 1 where R₃ is

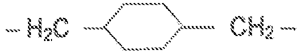

8. The container of claim 1 where R₃ is phenylene.

9. The container of claim 8 where the phenylene radical is derived from resorcinol, hydroquinone and/or catechol.

10. The container of claim 1 in which the polyether composition is aqueous based and in which the polyether (A) and/or (C) is grafted to a (meth)acrylic polymer.

## Patentansprüche

1. Behälter, der eine Lebensmittelkontaktoberfläche aufweist, wobei wenigstens ein Teil der Lebensmittelkontaktoberfläche mit einer Zusammensetzung beschichtet ist, die eine Polyetherzusammensetzung enthält, die sich von Benzoldimethanol ableitet und die wenigstens eines der folgenden Segmente aufweist: 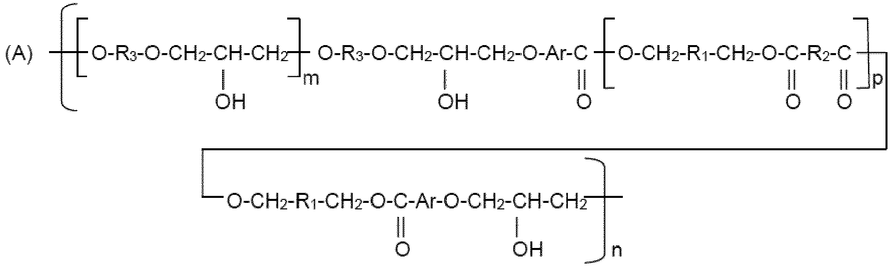 worin R₁ gleich Phenylen ist, R₂ ein gesättigter aliphatischer Rest ist, R₃ ist ein (cyclo)aliphatischer Rest, ein Arylenrest oder ein Alkarylenrest ist, m gleich 0 bis 12 ist, n gleich 1 bis 12 ist und p kleiner als 5 ist, oder 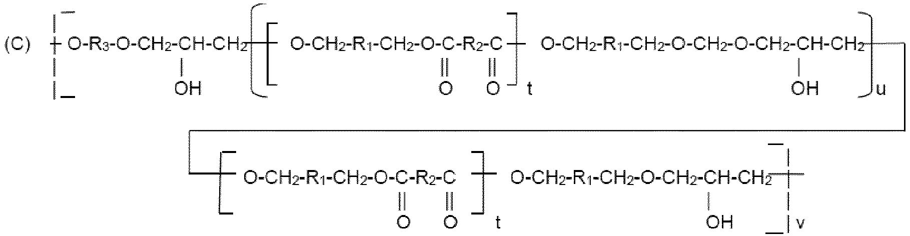 worin R₁ gleich Phenylen ist, R₂ ein gesättigter aliphatischer Rest ist, R₃ ein (cyclo)aliphatischer Rest, ein Arylenrest oder ein Alkarylenrest ist, t kleiner als 5 ist, u gleich 0 bis 12 ist und v gleich 1 bis 12 ist.

2. Behälter nach Anspruch 1, bei dem (A) mit Epoxygruppen oder phenolischen Hydroxylgruppen terminiert ist.

3. Behälter nach Anspruch 1, in dem (C) mit Epoxygruppen oder phenolischen Hydroxylgruppen terminiert ist.

4. Behälter nach Anspruch 1, worin Ar gleich Phenylen ist.

5. Behälter nach Anspruch 1, worin R₂ ein linearer Alkylenrest mit 4 bis 12 Kohlenstoffatomen ist.

6. Behälter nach Anspruch 1, worin p gleich 1 oder 2 ist.

7. Behälter nach Anspruch 1, worin R₃ 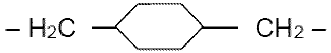 ist.

8. Behälter nach Anspruch 1, worin R₃ gleich Phenylen ist.

9. Behälter nach Anspruch 8, bei dem der Phenylenrest sich von Resorcin, Hydrochinon und/oder Catechol ableitet.

10. Behälter nach Anspruch 1, in dem die Polyetherzusammensetzung wasserbasiert ist und in der der Polyether (A) und/oder (C) auf ein (Meth)acrylpolymer gepfropft ist.

## Revendications

1. Récipient comportant une surface de contact alimentaire, dans lequel au moins une partie de la surface de contact alimentaire est revêtue d'une composition comprenant une composition de polyéther obtenue à partir de benzène-diméthanol, contenant au moins un des segments (A) et (C) suivants : 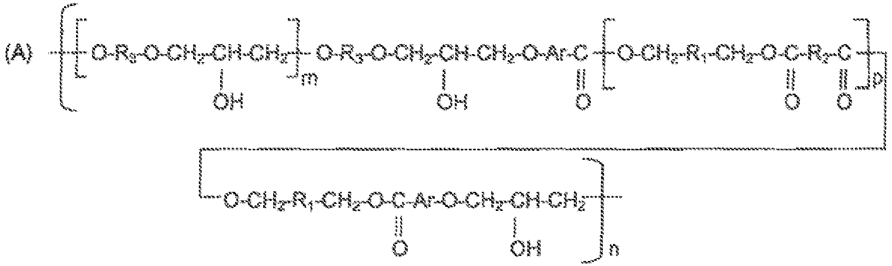 où R₁ représente un groupe phénylène, R₂ représente un groupe aliphatique saturé, R₃ représente un groupe (cyclo)aliphatique, un groupe arène-diyle ou un groupe alkylarène-diyle, l'indice m vaut de 0 à 12, l'indice n vaut de 1 à 12 et l'indice p est inférieur à 5,
et 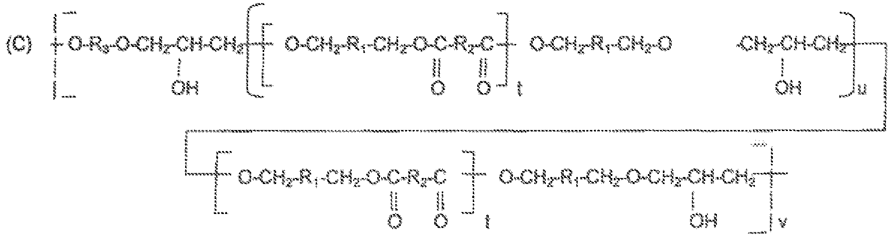 où R₁ représente un groupe phénylène, R₂ représente un groupe aliphatique saturé, R₃ représente un groupe (cyclo)aliphatique, un groupe arène-diyle ou un groupe alkylarène-diyle, l'indice t est inférieur à 5, l'indice u vaut de 0 à 12 et l'indice v vaut de 1 à 12.

2. Récipient selon la revendication 1, dans lequel le segment (A) a pour terminaisons des groupes époxyde ou des groupes hydroxyle phénoliques.

3. Récipient selon la revendication 1, dans lequel le segment (C) a pour terminaisons des groupes époxyde ou des groupes hydroxyle phénoliques.

4. Récipient selon la revendication 1, où Ar représente un groupe phénylène.

5. Récipient selon la revendication 1, où R₂ représente un groupe alcane-diyle linéaire comportant de 4 à 12 atomes de carbone.

6. Récipient selon la revendication 1, où l'indice p vaut de 1 à 2.

7. Récipient selon la revendication 1, où R₃ représente le groupe de formule

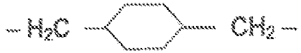

8. Récipient selon la revendication 1, où R₃ représente un groupe phénylène.

9. Récipient selon la revendication 8, où le groupe phénylène est obtenu à partir de résorcinol, d'hydroquinone et/ou de catéchol.

10. Récipient selon la revendication 1, dans lequel la composition de polyéther est une composition à base aqueuse, et le(s) polyéther(s) (A) et/ou (C) est ou sont greffé(s) sur un polymère (méth)acrylique.
